(19) Europäisches Patentamt / European Patent Office / Office européen des brevets

(11) **EP 4 375 665 A1**

(12) **EUROPEAN PATENT APPLICATION**
published in accordance with Art. 153(4) EPC

(43) Date of publication:
**29.05.2024 Bulletin 2024/22**

(21) Application number: **22845929.3**

(22) Date of filing: **19.07.2022**

(51) International Patent Classification (IPC):
**G01N 33/53** (2006.01)   **G01N 33/536** (2006.01)
**G01N 33/569** (2006.01)   **G01N 21/64** (2006.01)

(52) Cooperative Patent Classification (CPC):
**G01N 21/64; G01N 33/53; G01N 33/536; G01N 33/569**

(86) International application number:
**PCT/JP2022/028103**

(87) International publication number:
**WO 2023/002996 (26.01.2023 Gazette 2023/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.07.2021 JP 2021119181**

(71) Applicant: **Nanotis Corporation**
**Tokyo 151-0053 (JP)**

(72) Inventors:
• **MIYAZAKI, Jinsei**
**Osaka-shi, Osaka 578-0901 (JP)**
• **NAGAI, Yoko**
**Nagareyama-shi, Chiba 270-0141 (JP)**
• **NAGAMUNE, Teruyuki**
**Kawagoe-shi, Saitama 350-0808 (JP)**
• **KATO, Lisa**
**Tokyo 151-0063 (JP)**

(74) Representative: **Vossius & Partner**
**Patentanwälte Rechtsanwälte mbB**
**Siebertstrasse 3**
**81675 München (DE)**

(54) **METHOD FOR DETECTING VIRUS IN SPECIMEN, AND VIRUS DETECTION APPARATUS**

(57) The invention relates to a method for detecting a virus in a specimen, including: a step of preparing a test solution containing a specimen, a surfactant, and a fluorescently labeled anti-nucleocapsid protein antibody; a step of bringing the test solution into contact with a microelectrode and applying a voltage to the microelectrode so as to concentrate a fluorescently labeled anti-nucleocapsid protein antibody which is bound to a nucleocapsid protein of the virus in the specimen near the microelectrode; and a step of detecting the presence of the virus in the specimen by fluorescence observation, in which the nucleocapsid protein forms a complex with a nucleic acid.

Fig. 2

EP 4 375 665 A1

**Description**

Technical Field

The present invention relates to a method for detecting a virus in a specimen and a virus detection apparatus.

Background Art

The simple and highly sensitive detection of pathogenic viruses without the need for specialized biochemical analysis knowledge or operation techniques is a significant technical challenge that is required worldwide.

As disclosed in Non-Patent Literature 1, various measurement methods using antibodies based on immunochromatography have been developed. Immunochromatography can be used regardless of knowledge, technique, equipment, or environment. Therefore, for example, immunochromatography methods using antibodies that bind to influenza A and B viruses have contributed significantly to the rapid determination of influenza in clinical settings.

As described above, immunochromatography is a method that can easily be used to construct a system capable of convenient testing; however, there are technical hurdles in terms of improving sensitivity. For example, as disclosed in Non-Patent Literature 2, attempts have been made to improve sensitivity in immunochromatography by replacing labelling of antibodies in the mobile phase with fluorescent substances or enzymes that cause luminescence.

In addition, regarding influenza viruses, as disclosed in Non-Patent Literature 3, the success in improving the sensitivity by 100 to 1000 times will make it possible to test specimens using saliva instead of nasal secretion. Therefore, the burden on medical professionals and patients is expected to be reduced.

Here, the reaction between an antibody and an antigen is a reversible reaction, and a dissociation constant (Kd) specific to a monoclonal antibody is defined as shown in Equation 1 below.

Equation 1:

$$Kd = \frac{[Ab] \cdot [Ag]}{[Ab \cdot Ag]}$$

(In Equation 1, [Ab], [Ag], and [AbAg] denote the concentration of free antibody, antigen, and antibody-antigen conjugate, respectively.)

For simplicity, if [AbAg] = x and the initial antibody concentration and initial antigen concentration are $[Ab]_0$ and $[Ag]_0$, the following Equation 2 is obtained.

Equation 2:

$$x^2 - ([Ab]_0 + [Ag]_0 + Kd)x + [Ab]_0 \cdot [Ag]_0 = 0$$

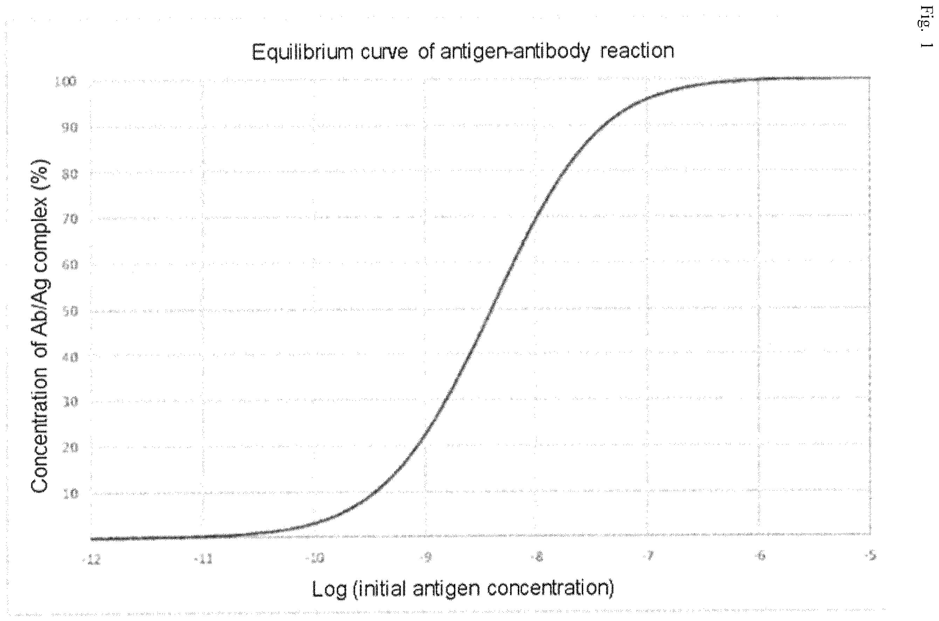

By solving Equation 2 and plotting [AbAg] against $[Ag]_0$, an S-shaped graph, as shown in FIG. 1, can be obtained. In FIG. 1, the y-axis corresponds to the output against the antigen-antibody complex concentration, and the x-axis corresponds to the concentration of the target substance in the specimen (scale is logarithmic). In the most typical state, when Kd = [Ab] = [Ag] = [AbAg], it is plotted at the center of the S-shape. Kd is unique to antibodies, and although it is theoretically possible to create monoclonal antibodies with as low a Kd as desired, the Kd values of available monoclonal antibodies are usually not specified. Among commercially available products, it is stated for one with the highest performance that ELISA detection was possible at a concentration of 1 mg/mL and a 3000-fold dilution, for example. Putting the molecular weight of the antibody in 150,000, the concentration of the antibody diluted 3000 times is $2 \times 10^{-9}$ M. Thus, it is assumed that the Kd of a monoclonal antibody is, at best, around $10^{-9}$ (even for one with a small value).

As is apparent from FIG. 1, when $[Ag]_0$ = around Kd, the measurement shows excellent sensitivity because y changes significantly with the increase or decrease of x, but in the region where x is small (the plateau region on the left), y is infinitely close to 0 regardless of the value of x. In this region, the value of [AbAg] is limitlessly close to 0, thus, when the output is amplified electrically, chemically, or optically, background noise that depends on non-specific adsorption or the like will be amplified simultaneously.

The present inventors have conducted research and development focusing on these characteristics of monoclonal antibodies based on the thought that rather than just amplifying the output sensitivity of a detection system based on antigen-antibody reactions, concentrating the antigen concentration to near the dissociation constant of the antibody is a practical method for increasing the sensitivity of the system.

Meanwhile, Patent Literature 1 suggests a method for separating a specific substance from a mixture in a specimen and labeling a specific substance with an antibody and the like based on the theory of dielectrophoresis.

Citation List

Patent Literature

Patent Literature 1: Japanese Patent Application Laid-Open No. 2001-165906

Non-Patent Literature

Non-Patent Literature 1: Nishijima, SCANS NEWS 2005 II
Non-Patent Literature 2: C. K. Lee et al, Journal of Clinical Virology 55 (2012) 239-243
Non-Patent Literature 3: A. Sueki et al, Clinica Chimica Acta 453, 71-74 (2016)

Summary of Invention

Technical Problem

Regarding infectious disease viruses such as the new coronavirus (SARS-CoV-2) and influenza virus, more sensitive measurement systems are required to detect viruses in specimens. An object of the present invention is to provide a novel measurement system that enables highly sensitive measurements without requiring specialized equipment, environment, knowledge, or technology.

Solution to Problem

The present inventors have conducted extensive studies and thus conceived of detecting a nucleocapsid protein (hereinafter also referred to as "NP") that is not affected by mutations to detect a virus since it is known that the spike proteins on the surfaces of influenza viruses and the new coronavirus (SARS-CoV-2) mutate frequently. This has led to the completion of the present invention.

The present invention is as follows.

[1] A method for detecting a virus in a specimen, comprising:

a step of bringing a test solution containing the specimen, a surfactant, and a fluorescently labeled anti-nucleocapsid protein antibody into contact with a microelectrode and applying an alternating-current voltage to the microelectrode so as to concentrate a fluorescently labeled anti-nucleocapsid protein antibody which is bound to a nucleocapsid protein of the virus in the specimen near the microelectrode; and
a step of detecting the presence of a virus in the specimen by fluorescence observation,
wherein the nucleocapsid protein forms a complex with a nucleic acid.

[2] The method according to [1], wherein the anti-nucleocapsid protein antibody is concentrated near the microelectrode by dielectrophoresis.
[3] The method according to [1] or [2], wherein an envelope of the virus in the specimen is chemically destroyed by the surfactant.
[4] The method according to any one of [1] to [3], which further comprises a step of electronically comparing fluorescence image changes in an aggregated state of fluorescence before and after voltage application and converting a difference image into a quantitative value or qualitative judgment result.
In the method according to any one of [1] to [4], a plurality of viruses may be simultaneously detected by simultaneously concentrating a plurality of anti-nucleocapsid protein antibodies fluorescently labeled with different wavelength regions.

[5] A virus detection apparatus, comprising:

a cell having microelectrodes at its bottom;
a power supply that applies an alternating-current voltage between the microelectrodes;
an image sensor,
means for electrically recording images; and
means for electronically comparing fluorescence image changes in an aggregated state of fluorescence before and after voltage application and converting a difference image into a quantitative value or qualitative judgment result,
wherein a fluorescently labeled anti-nucleocapsid protein antibody bound to a nucleocapsid protein of a virus in a specimen is concentrated near the microelectrodes so as to carry out fluorescence observation of fluorescence from an anti-nucleocapsid protein antibody. Here, the nucleocapsid protein forms a complex with a nucleic acid.

[6] The virus detection apparatus according to [5], wherein the fluorescently labeled anti-nucleocapsid protein antibody bound to a nucleocapsid protein of a virus is obtained by chemically destroying an envelope of the virus with a surfactant in a test solution containing a specimen, a surfactant, and a fluorescently labeled anti-nucleocapsid protein antibody.

[7] The virus detection apparatus according to [5] or [6], wherein a voltage is applied to the test solution such that the fluorescently labeled anti-nucleocapsid protein antibody bound to a nucleocapsid protein of a virus in a specimen is concentrated near the microelectrodes.

[8] The virus detection apparatus according to any one of [5] to [7], wherein the test solution is stirred by an electroosmotic flow. The virus detection apparatus may separately comprise a power supply that generates an electroosmotic flow, and the power supply that applies an alternating current voltage may also function as a power supply that generates an electroosmotic flow.

[9] The virus detection apparatus according to any one of [5] to [7], wherein protrusions that generate turbulent flow in a test solution are provided in the cell.

[10] The virus detection apparatus according to any one of [5] to [9], wherein a voltage, which can induce an electroosmotic flow and concentrate the fluorescently labeled anti-nucleocapsid protein antibody bound to a nucleocapsid protein of a virus in a specimen, is applied.

[11] The virus detection apparatus according to any one of [5] to [10], wherein the image sensor is a color sensor with a built-in color filter.

[12] The virus detection apparatus according to any one of [5] to [11], which is an apparatus for detecting a plurality of viruses simultaneously, wherein a plurality of anti-nucleocapsid protein antibodies fluorescently labeled with different wavelength regions are simultaneously concentrated.

The present invention may be the following embodiment.

[13] A method for detecting a virus in a specimen or a virus detection apparatus,

in the method according to any one of [1] to [4] or the virus detection apparatus according to any one of [5] to [12], wherein at least one of the microelectrodes and a combination of voltage and frequency of the applied voltage induces an electroosmotic flow to cause stirring of the test solution, and at least one of them can locally concentrate a nucleic acid/NP/labeled anti-P antibody complex by dielectrophoresis, and
wherein it is possible to simultaneously cause stirring by an electroosmotic flow and concentration by dielectrophoresis on the microelectrodes by applying an alternating current with voltage and frequency suitable for stirring the test solution by inducing an electroosmotic flow and an alternating current suitable for locally concentrating a nucleic acid/NP/labeled anti-P antibody complex by dielectrophoresis to one microelectrode, and/or the image sensor used is a color sensor with a built-in color filter, a plurality of anti-virus NP antibodies coexist, and an emission wavelength region of each antibody is independent to the extent that it can be evaluated independently using a combination of the image sensor and software, making it possible to simultaneously detect a plurality of different viruses.

Advantageous Effects of Invention

According to the present invention, highly sensitive measurements can be performed without requiring specialized equipment, environment, knowledge, and technology.

Brief Description of Drawings

FIG. 1 shows the equilibrium curve of the antigen-antibody reaction obtained by solving Equation 2 and plotting [AbAg] against $[Ag]_0$.

FIG. 2 shows a conceptual diagram of the inventive principle related to the NANOTIS method.

FIG. 3 shows a fluorescence spectrum of the Qd585-labeled anti-InflA-NP antibody (excitation: 350 nm).

FIG. 4 shows the shapes of microelectrodes for performing maskless photolithography. The gap between microelectrodes is 5 $\mu$m.

FIG. 5 shows the state of graphite distribution when a voltage is applied to the microelectrodes in FIG. 4 in non-ionized water.

FIG. 6A shows still images of microscopic images in Example 1 cut out from a video at each number of seconds.

FIG. 6B shows still images of microscopic images in Example 1 cut out from a video at each number of seconds.

FIG. 6C shows still images of microscopic images in Example 1 cut out from a video at each number of seconds.

FIG. 7 shows a graph in which the quantitative values and time shown in FIG. 6 are plotted as a numerical representation of dielectrophoresis images.

FIG. 8 shows a still image cut out immediately after voltage application in Example 2.

FIG. 9 shows a still image cut out 15 seconds after voltage application in Example 2.

FIG. 10 shows an example of the cell of Embodiment 1.

FIG. 11 shows an example of a cell provided with protrusions that generate turbulent flow according to Embodiment 1.

FIG. 12 shows a schematic diagram of Embodiment 2.

FIG. 13 shows fluorescence spectra of three types of quantum dots (Qd525, Qd585, Qd655). Fluorescence intensities were normalized to a maximum of 100, respectively.

Description of Embodiments

Embodiments of the present invention will be described in detail below. The present invention is not limited to the following embodiments, and can be implemented with various modifications within the scope of the gist of the present invention.

An embodiment of the present invention relates to a method for detecting a virus in a specimen, including:

a step of bringing a test solution containing the specimen, a surfactant, and a fluorescently labeled anti-nucleocapsid protein antibody into contact with a microelectrode and applying an alternating-current voltage to the microelectrode so as to concentrate a fluorescently labeled anti-nucleocapsid protein antibody which is bound to a nucleocapsid protein of the virus in the specimen near the microelectrode; and

a step of detecting the presence of a virus in the specimen by fluorescence observation, wherein the nucleocapsid protein forms a complex with a nucleic acid.

Another embodiment of the present invention relates to a virus detection apparatus, including:

a cell having microelectrodes at its bottom;

a power supply that applies an alternating-current voltage between the microelectrodes;

an image sensor,

means for electrically recording images; and

means for electronically comparing fluorescence image changes in an aggregated state of fluorescence before and after voltage application and converting a difference image into a quantitative value or qualitative judgment result, wherein a fluorescently labeled anti-nucleocapsid protein antibody bound to a nucleocapsid protein of a virus in a specimen is concentrated near the microelectrodes so as to carry out fluorescence observation of fluorescence from an anti-nucleocapsid protein antibody.

According to the present invention, the presence of a virus in a specimen contained in a test solution can be detected using a virus detection apparatus in one embodiment of the present invention.

Here, detecting the presence of a virus means confirming the presence or absence of a virus in a specimen whose presence or absence of a virus is desired to be confirmed.

The present inventors consider that the method for detecting a virus in a specimen and the virus detection apparatus of the present invention are based on the following theory.

The force ($F_{DEP}$) applied to a particle during dielectrophoresis is given by Equation 3 below.

Equation 3:

$$F_{DEP} = 2\pi r^3 \varepsilon_m \mathrm{Re}[K(\omega)]\nabla E^2$$

(In Equation 3, r denotes the radius of the particle, and VE denotes the non-uniformity of the electric field on both sides of the particle.)

As is apparent from Equation 3, the force applied to the particle is proportional to the cube of the radius of the particle.

Bacteria have particle diameters of several micrometers, and dielectrophoresis is likely to occur, whereas viruses, such as influenza viruses, have particle diameters of about 0.1 micrometers; thus, they can only obtain an acceleration of 1/1000.

Here, since the particle diameter of NP is estimated to be a little less than about 10 nm, it is even less than 1/1000 of $F_{DEP}$ in the case of a virus. Therefore, it is challenging to concentrate NP directly by dielectrophoresis. The present inventors have already considered using particles, such as graphite, which are highly susceptible to dielectrophoresis as carriers; however, this requires a process of selectively immobilizing NPs on the carriers, making the reaction system complicated.

In the present invention, an alternating-current voltage is applied to the microelectrodes used for applying voltage such that the electric field between the electrodes is from 0.1 to 50 MV/m. The electric field is determined by (voltage between peaks of applied alternating current (V))/(distance between electrodes (m)). Practically, the gap between the electrodes is from 0.2 to 100 μm, preferably from 1 to 10 μm. At this time, the alternating current voltage applied is, for example, from 1 to 50 V, and the electric field strength is from 0.1 to 50 MV/m.

A virus to be detected in the present invention is not particularly limited as long as it is a virus whose NP forms a complex with a nucleic acid but is preferably an enveloped virus.

Examples of an enveloped virus include, but are not limited to, coronaviruses (COVID-19 and conventional types), influenza viruses, herpes viruses, and rubella viruses.

Compared with the enveloped virus, the spike protein on the surface frequently mutates, as in influenza virus and new coronavirus (SARS-CoV-2). Therefore, rather than targeting the spike protein, the present invention targets the nucleocapsid protein and utilizes it for virus detection.

The nucleic acid may be DNA or RNA, depending on the target virus, but RNA is preferable.

Furthermore, the nucleocapsid protein is known as a protein that binds to the DNA or RNA of a target virus.

In the present invention, it is preferable to prepare a test solution containing a specimen, a surfactant, and a fluorescently labeled anti-nucleocapsid protein antibody.

The method for detecting a virus in a specimen in an embodiment of the present invention may comprises a step of preparing a test solution containing a specimen, a surfactant, and a fluorescently labeled anti-nucleocapsid protein antibody.

The test solution may be prepared in a cell having microelectrodes at its bottom, which is provided to the virus detection apparatus, or in a sample from which a specimen is collected. The test solution may also be prepared after a specimen is collected such that the test solution contains a surfactant and a fluorescently labeled anti-nucleocapsid protein antibody.

The preparation of the test solution can be appropriately set depending on the embodiment of the method for detecting a virus.

The material, shape, and the like of the cell included in the virus detection apparatus are not particularly limited and can be set appropriately as long as it is a cell that allows the test solution to pass through the electrode gap by capillary action and is transparent enough to observe fluorescence.

The walls of the cell may be coated with a surfactant and/or a fluorescently labeled anti-nucleocapsid protein antibody.

The specimen is not particularly limited as long as it is possible to eventually confirm the presence of a virus in the specimen; however, examples include samples from living organisms. Samples collected from food, and those present in buildings such as factories, schools, and hospitals may also be used as specimens.

Desired examples of samples from living organisms include, but are not particularly limited, human saliva or nasal swabs, and nasal secretions. The obtained sample from a living organism may be directly used as a detection target, or a sample diluted, suspended, or dissolved in a solvent such as water or alcohol may be used. The solvent such as water or alcohol may contain a surfactant.

The surfactant may be any substance as long as it can destroy the envelope of the virus, and examples thereof include Triton X-100, although it is not particularly limited.

Once a test solution is prepared, it will be described below as containing a virus to be detected, but even when it does not have a virus, the reactions and steps proceed in the same way.

The envelope of the virus in the specimen is chemically destroyed using a surfactant, and the nucleic acid/NP complex is leaked out of the virus. In the preparation step, a test solution containing a specimen, a surfactant, and a fluorescently labeled anti-nucleocapsid protein antibody is prepared. It is also possible to mix a specimen and a surfactant in advance to allow a nucleic acid/NP complex to leak outside the virus. Then, an anti-nucleocapsid protein antibody may be mixed with the resulting solution. It is also possible to perform an operation of concentrating the complex on the solution in which a nucleic acid/NP complex has leaked outside the virus and then mix an anti-nucleocapsid protein antibody therewith.

Nucleic acids are huge molecules similar to viruses. It was initially challenging to concentrate molecules of several nanometers, such as NPs and NP/antibody complexes, by dielectrophoresis. However, the present inventors conceived of using a nucleic acid as a target for dielectrophorésis as an alternative to the carrier mentioned above, such as graphite, leading to the present invention.

In the case of influenza, approximately 1000 molecules of NP are adsorbed per molecule of RNA (Biochem.J. (1983) 211, 281). As a result, NP is eluted into the test solution as a nucleic acid/NP complex in which approximately 1000 molecules of NP are firmly adsorbed to one molecule of nucleic acid (in the case of influenza and the new coronavirus, single-stranded RNA).

Although it is challenging to perform dielectrophoresis on NPs directly, the present inventors have confirmed that dielectrophoretic concentration can be performed under practical conditions by using nucleic acids as a carrier, as shown in the following Examples.

A polyclonal antibody may be used as an anti-nucleocapsid protein antibody, but a monoclonal antibody is preferable. In addition, affinity can be maintained by performing fluorescent labeling via the sugar chain in the Fc region using an antibody having a sugar chain.

A monoclonal antibody can be produced by a conventionally known method. An antibody produced by a known method or a commercially available antibody can be used.

Since the origin of the antibody is not particularly limited, examples thereof include mammals and may also be experimental animals. Specifically, antibodies derived from mice, rats, rabbits, camels, and the like can be used. The antibody may be a human antibody, a chimeric antibody, a humanized antibody, or the like.

Antibodies have classes such as IgG, IgA, IgM, IgD, and IgE. IgG or IgM may be preferably used, but there is no particular limitation. For example, even when IgG is used, subclasses such as IgG1 to IgG4 are not particularly limited.

Fragments of these antibodies described above may be used instead of anti-nucleocapsid protein antibodies.

The anti-nucleocapsid protein antibody is fluorescently labeled. As the fluorescent substance used for fluorescent labeling, a substance that satisfies some or all of the following requirements is preferably used.

- The excitation wavelength is long, preferably 350 nm or more, and more preferably 400 nm or more.
- The fluorescence wavelength is short, preferably 1500 nm or less.
- The extinction coefficient is high, preferably, the molar extinction coefficient is 10,000 or more.
- The quantum yield is high, preferably 0.1 or more.
- In a case in which a plurality of fluorescent substances are used, at a wavelength for exciting one fluorescent substance, another fluorescent substance is not excited, and when the fluorescence of another fluorescent substance is measured, the fluorescence of the one fluorescent substance does not overlap. In this case, to allow the fluorescence of one fluorescent substance to promote the excitation of another fluorescent substance efficiently, it is preferable that the peak of the fluorescence spectrum of the one fluorescent substance is sufficiently close to the peak of the excitation spectrum of another fluorescent substance. Further, it is preferable that the fluorescence spectrum of one fluorescent substance is sufficiently narrow and the excitation spectrum of another fluorescent substance is sufficiently broad. It is more preferable that the difference between the positions of the excitation spectrum peak and the fluorescence spectrum peak (Stokes shift) is sufficiently large.

Further, it is preferable that fluorescent substances satisfy some or all of the following requirements.

- The water solubility of an antibody to be labeled is not inhibited.
- The specific binding between NP and an antibody is not inhibited after labeling.
- Labeling does not cause aggregation of unlabeled material and/or enhancement of light scattering.

The fluorescent substance is preferably a quantum dot.

For fluorescent labeling, fluorescent particles made of a semiconductor having a diameter of several nanometers (nm), commonly called quantum dots, have an extinction coefficient and quantum yield several tens of times higher than organic dyes. Their fluorescence is strong, and half-width is narrow, making it easy to obtain high performance as a system. Another great advantage is that by using a method of binding a quantum dot to a sugar chain of an antibody, it is possible to completely avoid the reduction in affinity due to chemical manipulation of the Fv region, which may occur in some cases.

A test solution may be stirred by an electroosmotic flow. A test solution may also be stirred by providing protrusions that generate a turbulent flow in the test solution in a cell.

Stirring of the test solution facilitates the elution of a nucleic acid/NP complex in the test solution and is helpful for concentration by dielectrophoresis.

In the case of stirring the test solution by the electroosmotic flow, an alternating current voltage, which can induce the electroosmotic flow and concentrate a fluorescently labeled anti-nucleocapsid protein antibody bound to the nucleocapsid protein of a virus in a specimen, may be applied.

The shapes and locations of protrusions that generate the turbulent flow can be set appropriately, considering the balance between the effect of stirring the test solution and the flow rate.

According to the present invention, an anti-nucleocapsid protein antibody that specifically recognizes NP is fluorescently labeled to detect the presence of a virus in a specimen by fluorescence observation.

Since the anti-nucleocapsid protein antibody recognizes NP, it binds to NP of a complex of nucleic acid and NP such that the nucleic acid as a carrier is concentrated by a microelectrode due to dielectrophoresis.

In the present invention, a test solution is brought into contact with a microelectrode, and an alternating-current voltage is applied to the microelectrode so as to concentrate a fluorescently labeled anti-nucleocapsid protein antibody which is bound to a nucleocapsid protein of a virus in a specimen near the microelectrode.

The virus detection apparatus comprises a power supply that applies an alternating current voltage between microelectrodes. The power supply is not particularly limited, and any power supply capable of applying an alternating current voltage to yield an electric field between electrodes of from 1 to 10 MV/m may be used.

The power supply is not particularly limited, and any power supply that can secure the desired frequency and voltage may be used. For example, MAX038 from Maxim Integrated may be used.

In the present invention, the presence of a virus in a specimen is detected by fluorescence observation.

Fluorescence of an anti-nucleocapsid protein antibody concentrated near a microelectrode may be observed. Fluorescence of an anti-nucleocapsid protein antibody that is being concentrated near a microelectrode may also be observed.

It has been found that by labeling a monoclonal antibody that specifically binds to NP with a fluorescent substance (desirably a quantum dot) and allowing the labeled monoclonal antibody to be present such that a nucleic acid/NP/labeled antibody complex is concentrated near a microelectrode and the movement thereof can be captured as the movement of a fluorescent light spot. This allows virus detection in the present invention. In other words, in addition to the fact that the NP/RNA complex is a larger particle than the NP, one contributing factor is that the NP/RNA complex further forms a matrix via the antibody, which further accelerates dielectric concentration.

For fluorescence observation, the virus detection apparatus includes:

> an image sensor; and
> means for electrically recording images.

With the virus detection apparatus, a fluorescently labeled anti-nucleocapsid protein antibody bound to a nucleocapsid protein of a virus in a specimen is concentrated near the microelectrodes so as to carry out fluorescence observation of fluorescence from an anti-nucleocapsid protein antibody. In this case, it is possible to electronically compare fluorescence image changes in an aggregated state of fluorescence before and after voltage application and convert a difference image into a quantitative value or qualitative judgment result for fluorescence observation.

Therefore, the virus detection apparatus may further include the means for electronically comparing fluorescence image changes in an aggregated state of fluorescence before and after voltage application and converting a difference image into a quantitative value or qualitative judgment result.

The following methods may be selected appropriately for fluorescence observation with the image sensor:

> (1) measuring fluorescence by placing a transparent insulating film on an image sensor;
> (2) installing an image sensor on the outside of the bottom of a detection cell to measure fluorescence;
> (3) providing a slit with the same anisotropy as an electrode on an image sensor to measure fluorescence, as the electrode has anisotropy in the X and Y directions; and
> (4) installing an image sensor on the side of a detection cell and irradiating excitation light from the bottom of the detection cell to measure fluorescence.

The image sensor may be a color sensor with a built-in color filter. As an example, SWIFT's Digital Microscope Eyepiece (electronic eyepiece, biological microscope compatible, 5 million pixels, 5MP, HD, USB 2.0) is already equipped with an electronic circuit and may be used suitably.

Conditions for fluorescence observation may also be set appropriately. The excitation light generally has a wavelength of from 300 to 600 nm, preferably from 350 nm or more, and more preferably 400 nm or more.

For fluorescence observation, it is possible to perform measurement using a filter + an optical sensor (photodiode, phototransistor) or to take an image of the illuminated state with a smartphone image sensor and compare the image with a known electrode shape for image recognition, thereby extracting only the illuminated part, which makes it possible to enhance contrast and perform more accurate detection.

Furthermore, as means for electrically recording images, a hard disk (or SSD) or the like may be used, and image data may also be stored on a recording disk, or an external storage device such as a USB memory may be used.

Algorithms and programmed versions of algorithms known as libraries may be used as means for electronically comparing the fluorescence image changes in the aggregated state of fluorescence before and after voltage application and converting the difference image into a quantitative value or qualitative judgment result. One example is ImageJ, which is popular as open-source software. By using ImageJ, automation is possible, and it is also possible to automate the entire series of operations and analysis. The calculation between images may be performed using any calculation method other than the difference, such as exclusive OR (XOR), which makes it clear that the images differ from the base image.

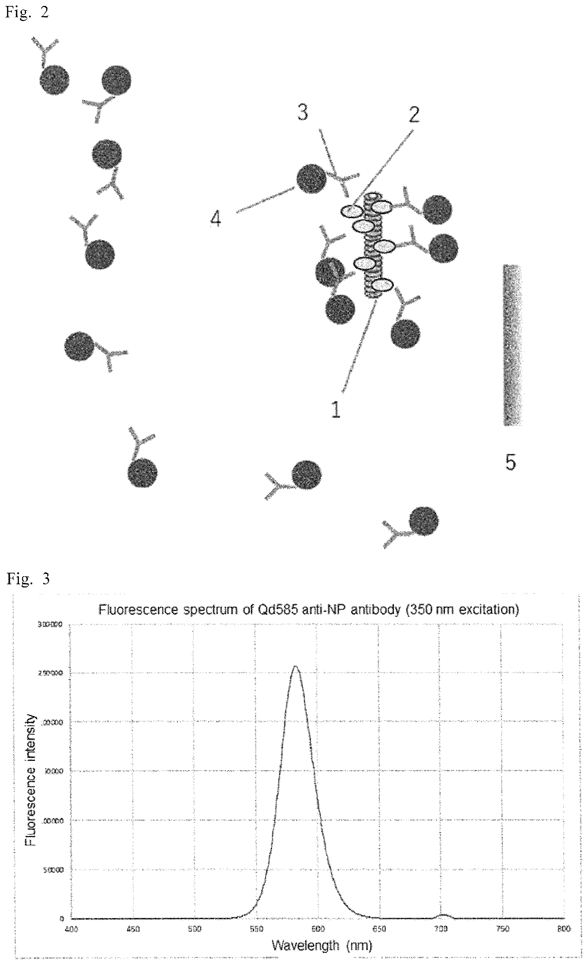

Hereinafter, this method will be referred to as a NANOTIS method stands for "Nucleic Acid Navigated Optically Traceable Immuno-Sensing" method for ease of explanation. FIG. 2 shows a conceptual diagram of the inventive principle related to the NANOTIS method.

In FIG. 2, 1 denotes a nucleic acid, which may be DNA or RNA, depending on the virus to be detected. A nucleocapsid protein (NP) 2 is bound to a nucleic acid 1, forming a nucleic acid/NP complex. The nucleic acid/NP complex is eluted from a virus by a surfactant and thus present in a test solution. When an anti-nucleocapsid protein antibody (anti-NP antibody) 3 is present, a nucleic acid/NP/anti-NP antibody complex is formed. The anti-NP antibody 3 is fluorescently labeled with a fluorescent substance 4. By applying an alternating current voltage, a nucleic acid/NP/anti-NP antibody complex is concentrated near a microelectrode 5, and by observing the fluorescence of the fluorescent substance labeled on the anti-NP antibody, the presence of the virus in the specimen is confirmed.

By using the NANOTIS method, the following benefits can be simultaneously obtained.

• NP, which is difficult to concentrate using the dielectrophoresis method, becomes possible to be concentrated.

• By using the nucleic acid endogenously present in the virus as a carrier, it is possible to avoid creating a complicated system in which a carrier is added from the outside. This suppress the variations that tend to occur in a heterogeneous system without.

• Sample pretreatment is simplified since there is no need to add a carrier.

• Since there is no need to search for an antibody pair with different epitopes as is used in a sandwich reaction, the only antibody required for detection is one type of anti-NP antibody, which simplifies the system and allows a new detection target to be handled in a shorter time.

• When no pair of antibodies undergoes a sandwich reaction, but an antibody with an outstandingly low dissociation constant is pickable, which induce effect to increase sensitivity.

· When NP is handled alone, there are detection noise factors such as aggregation, gelation, and adsorption to the solid phase surface. However, since NP is stabilized by nucleic acid in the nucleic acid/NP complex state, these noise factors may be eliminated.

· Approximately 1000 molecules of NP are bound to one nucleic acid molecule (in influenza, it consists of eight segmented single-stranded RNA), making it easy to obtain sensitivity.

· Viruses are 0.1 $\mu$m and cannot be detected with an optical microscope; however, it is possible to capture the movements of nucleic acids, NPs, and labeled antibodies by regarding them as fluorescent light spots.

· In this detection, nucleic acids only contribute to dielectrophoresis as carriers, while the contribution of NP varies depending on the virus species. In addition, the specificity of detection may be ensured by the specificity of NP and anti-NP antibody (it has been confirmed that the antibody per se has no cross-reactivity with NP of influenza B virus and other viruses).

· Since the determination is based on the specificity of NP, which does not readily undergo mutation, it is also possible to detect spike protein variant viruses.

· Since the reaction involves one type of antigen and one type of antibody, high sensitivity may be achieved by adding a significant excess of a labeled antibody in advance. In a sandwich system using two types of antibodies, the detection range is affected by bias in the amounts (or concentrations) of the antibodies; thus, the simplicity of the reaction components facilitates the optimization of the entire reaction system.

According to Sasaki, Bunseki Kagaku, Vol.64, No1, 1 (2015), as long as conditions such as the size of the electrodes, the size between the electrodes, the applied voltage, and the frequency are met, the phenomena in which the solvent itself moves, such as the AC electroosmotic flow and the AC thermodynamic current, can occur. The present inventors further confirmed the phenomenon that nucleic acids, NPs, and labeled antibodies gather by dielectrophoresis while the solvent itself moves depending on the shape and material of the electrode. It is not always necessary to precisely classify the electrical effects caused by multiple factors. The NANOTIS method of the present invention is intended to apply an alternating current with non-uniform electric field density between electrodes and, as a result, accumulate and detect target nucleic acids, NPs, and labeled antibodies near the electrodes. The present inventors have also confirmed that it is also possible to efficiently use the solvent for stirring in the Z direction by actively inducing movement of the solvent itself. In some cases, a frequency optimal for dielectrophoresis and a frequency optimal for AC conduction phenomenon may be applied simultaneously. Alternatively, an electrode for dielectrophoresis and an electrode for stirring may be provided simultaneously in the same space, and frequencies optimal for each purpose may be applied.

According to the NANOTIS method, the virus detection apparatus of the present invention may be an apparatus for detecting a plurality of viruses simultaneously, which is a virus detection apparatus in which a plurality of anti-nucleocapsid protein antibodies fluorescently labeled with different wavelength regions are simultaneously concentrated, and the method for detecting a virus of the present invention may be a method for detecting a plurality of viruses simultaneously.

Further, the description of the virus detection apparatus of the present invention and the description of the method for detecting a virus of the present invention may complement each other. In other words, the description of the virus detection apparatus of the present invention can also be applied to the method for detecting a virus of the present invention, and the description of the method for detecting a virus of the present invention can also be applied to the virus detection apparatus of the present invention.

Examples

The present invention will be further explained below with reference to Examples, but the present invention is not limited to the following Examples.

The materials and apparatus for a test solution used in the following Examples are as follows.

(Materials for Test Solution)

Substance to be detected (virus): Influenza A virus inactivated by gamma rays was obtained from Bio-Rad Laboratories, Inc. The product number is PIP021, and the antigenicity is H1N1.

Antibody: Anti-influenza A virus NP monoclonal antibody (FIA-2121) was obtained from BIO MATRIX RESEARCH, INC. The manufacturer has confirmed that it binds equally to the NPs of 15 types of mutant influenza A viruses and does not cross-react to the NP of influenza B virus.

Fluorescently labeled antibody (labeled antibody): Using the SiteClick "Qdot" 585 Antibody Labeling Kit (manufactured by Thermo Fisher Scientific), the sugar chains in the Fc region of the antibody were labeled with quantum dots according to the protocol provided by the company. FIG. 3 shows the fluorescence spectrum of the antibody.

Surfactant: Triton X-100 (Sigma-Aldrich) was used to disrupt the viral envelope.

(Apparatus)

Conductivity meter: EC-33B (HORIBA Advanced Techno, Co., Ltd.)
Fluorescence microscope: Leica DFC360FX
Alternating current generator: KKmoon FY6800 digital function generator
Oscilloscope: Hewlett-Packard 54602B

Example 1

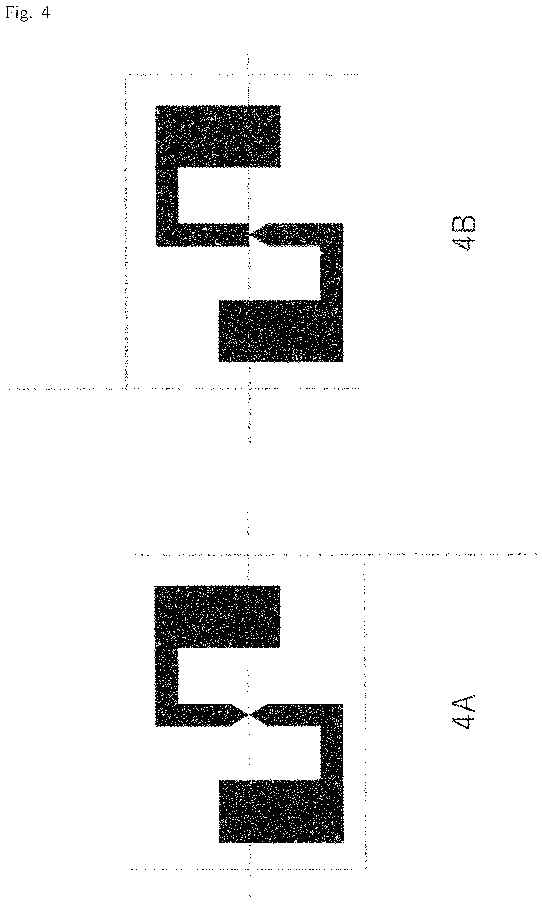

Two types of microelectrodes were formed using chromium photolithography on a glass substrate in the shape shown in FIG. 4. A silicone sheet (0.05 mm thick) with a $2 \times 3$ mm hole was pasted around the electrode gap, thereby forming a liquid reservoir (cell).

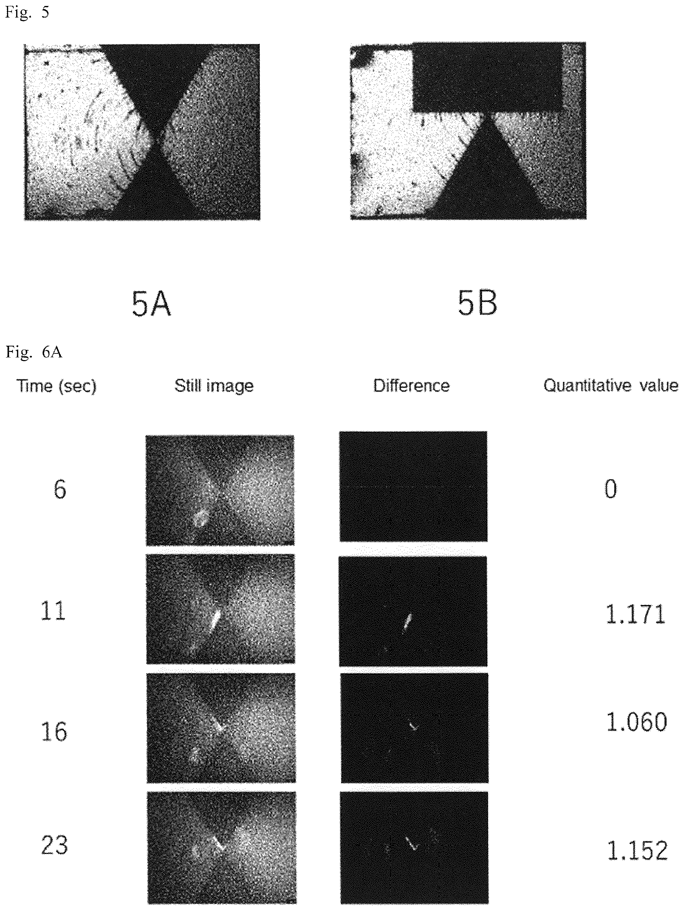

To confirm the distribution of the electric field gathered at the electrode, graphite having an average particle size of $2 \ \mu m$ dispersed in non-ionized water (water sampled from Merck Millipore's MilliQ) was placed in a liquid reservoir, and a 20 V, 30 kHz alternating current was applied for 30 seconds. The distribution state of graphite after the application is shown in FIG. 5.

To idealize the conductivity of the test solution, the virus, surfactant, and labeled antibody solutions were each replaced with nonionized water three times by centrifugal filtration using Nanosep (registered trademark) (fraction: 300k, Pall Corporation).

The concentrations of the virus, surfactant, and labeled antibody in the test solution were adjusted to the following concentrations. After confirming that the test solution had come to rest while observing with a fluorescence microscope, an alternating-current voltage was applied. The time from mixing to voltage application was within 2 minutes. Electrode oscilloscopes were connected in parallel to check the frequency and voltage constantly.

Virus concentration: 0.56 mg/mL
Surfactant concentration: 0.3%
Labeled antibody concentration: 75 nM
The conductivity of the test solution was 240 $\mu$S/cm.

Specifically, the details are as follows.

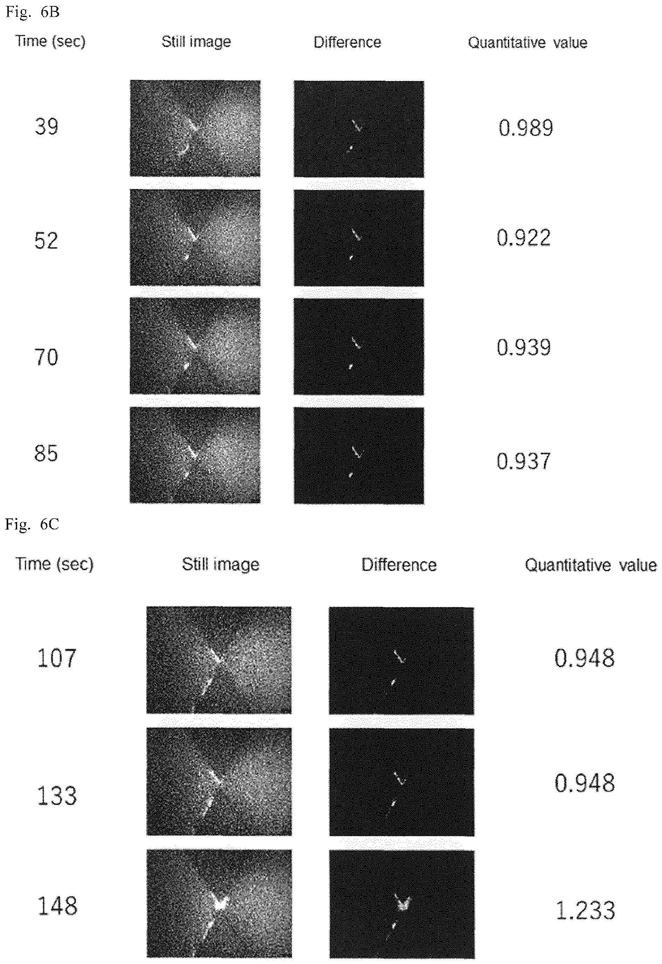

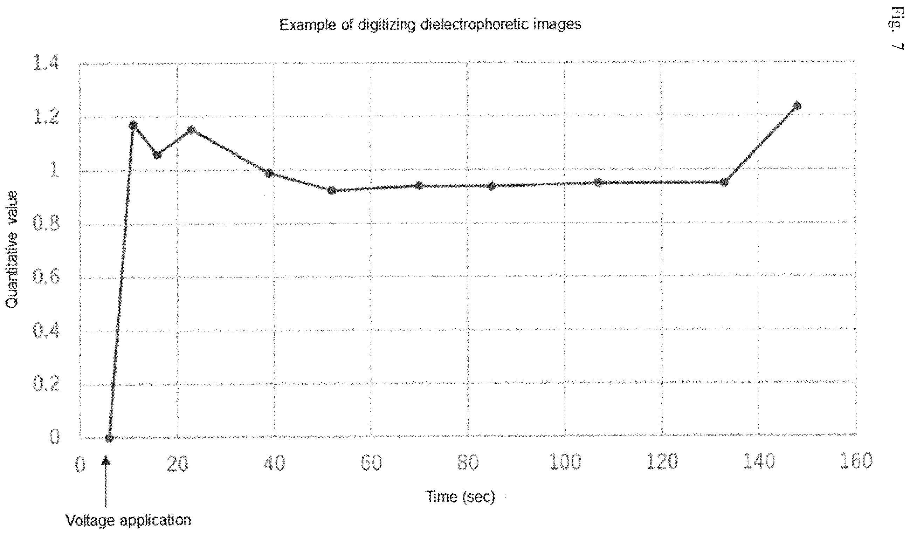

The prepared liquid mixture of virus and labeled antibody (5 $\mu$L) was placed in the liquid reservoir of the microelectrode, which had been placed on a fluorescence microscope in advance. Fluorescence microscopy recording was started, and the surfactant solution was added dropwise to the final concentration above. After about 15 seconds, it was confirmed that the fluctuations in the solution had subsided, and saving videos of the microscopic images was started. When an alternating-current voltage of 20 V, 30 kHz was applied after 8 seconds, with the start of storage as 0 seconds, it was possible to confirm that the entire liquid was immediately moving in a spiral, and light spots were gathering during this movement. Microscope images were recorded up to 148 seconds. FIG. 6 shows still images cut out from the video image at each number of seconds. A still image at 6 seconds is used as a reference image, and a difference image between each still image and the reference image is created using ImageJ, which is shown as the difference in the third column. Analyze/Measure is also performed on these different images using ImageJ, and the average brightness of each pixel is shown as a quantitative value in the fourth column. FIG. 7 shows a plot of time and quantitative values.

In addition, an upright fluorescence microscope was assembled by installing an excitation light on an upright microscope. It was confirmed that the fluorescence observation mentioned above could be made by observing the light spot above the electrode. Finally, by applying a threshold to this quantitative value, the concentration of influenza virus can be changed to a pseudo-quantitative value, preferably in 5 to 7 steps, and at the same time can be displayed as a qualitative judgment value of negative or positive. More preferably, the saliva is placed in a diluent, thereby reducing the conductivity of the solvent. The diluent may be a mixture of sugar alcohol with low conductivity, such as D(-)mannitol and nonionized water, and it is more convenient if the diluent contains a labeled antibody and a surfactant in advance. It would be further preferable to automate all the steps of sucking this mixed sample into the detection cell by capillary action, starting video recording when the detection cell is set in a simple fluorescence microscope, applying an alternating current, processing the video using the above-described method after a specified time, and changing the processed data to the judgment value. Subtracting the manual operation time, the time from setting the detection cell to detection was less than 120 seconds. Compared to taking the usual 5 to 10 minutes to obtain results using immunochromatography, a significant speedup could be confirmed. Furthermore, due to conducting similar experiments with different virus concentrations, approximately 100 times higher sensitivity was achieved than in immunochromatography.

Example 2

Example 2 was carried out as in Example 1.

Only the electrode used was the Au electrode from BAS (comb-shaped; electrode width: 10 $\mu$m; electrode spacing: 5 $\mu$m). When searching was conducted widely from 1 kHz to 20 MHz, favorable dielectrophoresis was observed at 2 MHz.

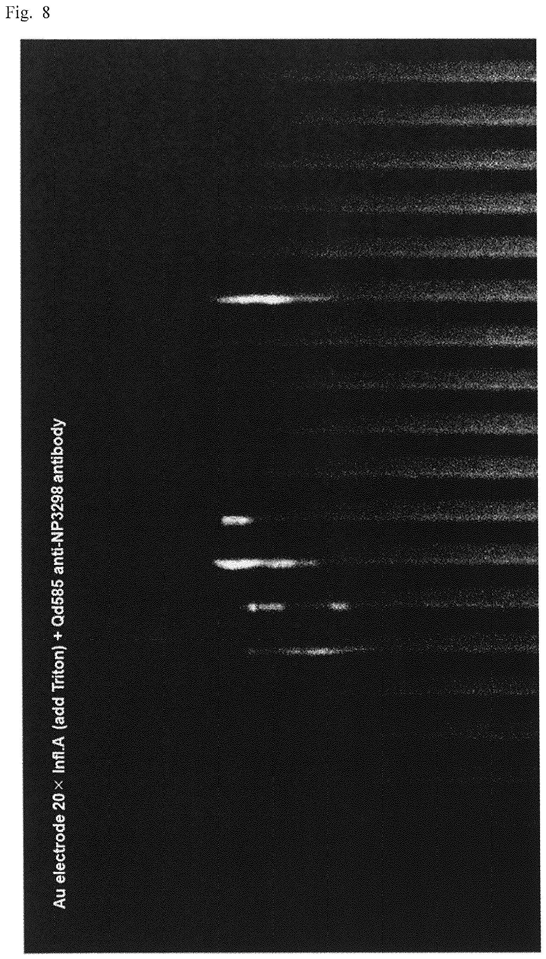

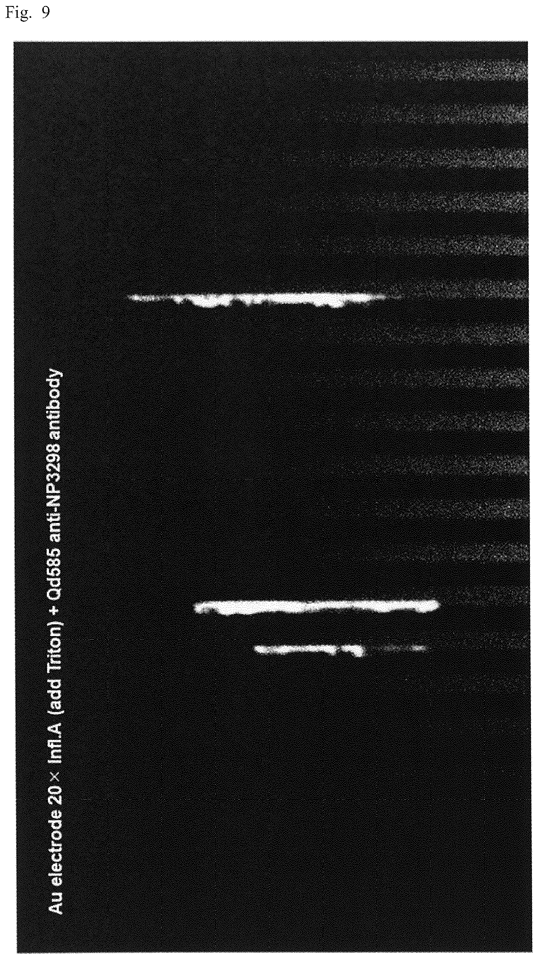

FIG. 8 shows a still image immediately after voltage application, and FIG. 9 shows a still image 15 seconds after voltage application.

It was observed in Example 2 that dielectrophoresis gathered in a higher frequency range than the microelectrode used in Example 1, and the accumulation rate was faster. It is speculated that this is influenced by the sharpness of the cross-section of the electrode edge and the conductivity of the electrode material. In addition, although these are the observation results under the conditions of this experiment when comparing experiments using both electrodes, the electroosmotic flow was more pronounced in the low-frequency region (from 1 to 10 kHz) using the ITO electrode. From the results, the electroosmotic flow and concentration by dielectrophoresis within one detection cell become possible by combining the shapes and materials of electrodes to create, for example, one detection cell equipped with the microelectrode used in Example 1 to which 1 to 100 kHz was applied and a comb-shaped Au electrode to which 100 kHz to 10 MHz was applied such that different electrodes can carry out stirring by the electroosmotic flow and concentration by dielectrophoresis or by applying a frequency band suitable for the electroosmotic flow and a frequency band suitable for dielectrophoresis sequentially or simultaneously to a detection cell equipped with only one type of Au electrode or microelectrode. In order to obtain a similar effect more easily, it is possible to create mechanical non-uniformity, such as unevenness, in the middle of the capillary flow path of the detection cell, and to stir the flow by making it turbulent in front of the detection portion is also possible.

Embodiment 1

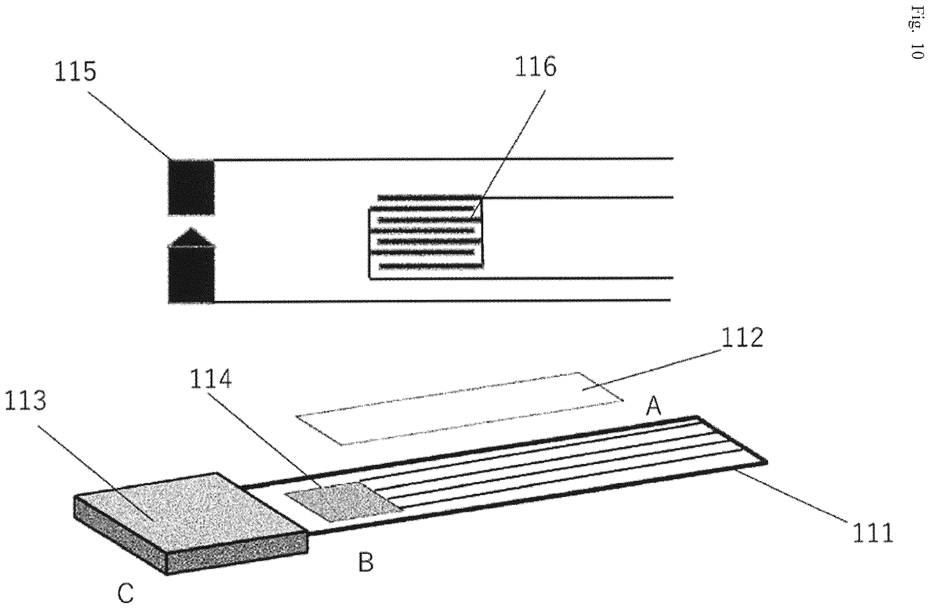

Based on Examples 1 and 2, an embodiment of a detection cell with an enhanced concentration effect is shown in FIG. 10. In Examples 1 and 2, the RNA/NP/labeled antibody complex, which is the object to be detected, was locally concentrated near the electrode from the test solution set in the liquid reservoir (2 × 3 × 0.05 mm; volume of the liquid reservoir: 0.3 $\mu$L) provided on the top of the microelectrode. Assuming that the specimen is saliva or nasal swab fluid (preferably saliva) when diluting it 2 to 10 times with a low-conductivity solvent containing a labeled antibody or surfactant, it is possible to easily collect from 100 to 1000 $\mu$L of the specimen. According to the present invention, the main focus is on how to concentrate a specimen with a low concentration to bring the antibody concentration close to the antibody divergence multiplier. Therefore, a preferred object of the present invention can be achieved by bringing more specimens into contact with a minute detection portion with a limited area. FIG. 10 shows an embodiment of a detection cell designed with this point in mind. In FIG. 10, 111 denotes a transparent substrate, and a composite electrode 114 is provided on the surface thereof. Describing 114 in more detail, it is provided with a microelectrode 115 for detection and an electrode 116 for generating an electroosmotic flow. A plurality of pairs of microelectrodes may be used to increase the collection efficiency. One pair may be used to confirm the effect more clearly. FIG. 10 shows a case with one pair of microelectrodes. Here, 115 is preferably made of highly conductive and corrosion-resistant Au, copper, or Pt, and 116 is preferably made of ITO, which efficiently generates an electroosmotic flow in the Examples; however, the same material may be used for convenience and lower cost of production. 112 is fixed to 111 with an appropriate spacing (preferably from 20 to 200 $\mu$m) to cause capillary action in the cover glass. 113 is a water absorption pad, which may be the same as that used at the end of immunochromatography.

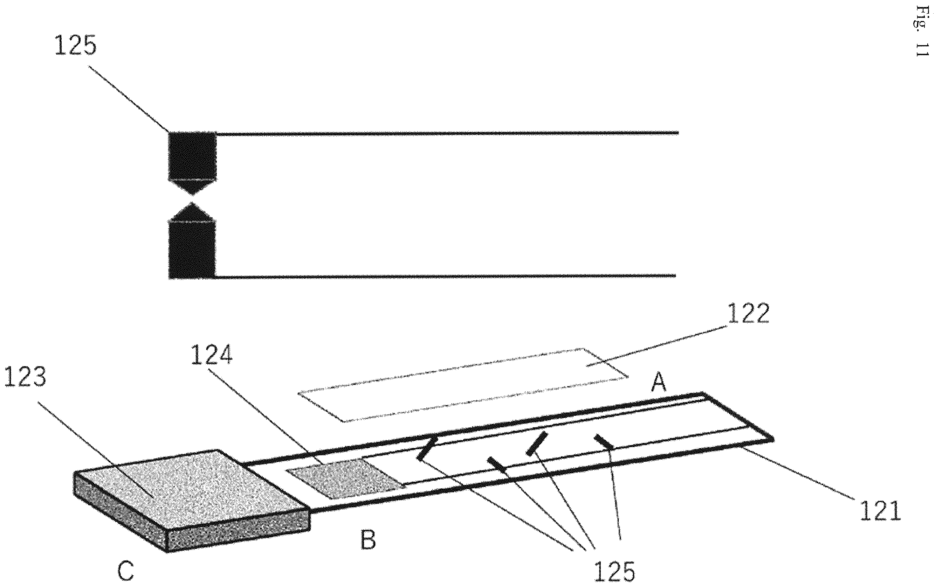

By setting the test solution at location A, it flows toward C (in the x direction) by capillary action, and at this time, it contacts the composite electrode 114 in region B. In B, stirring in the depth direction (z direction) is performed by the electroosmotic flow. Furthermore, the concentration in the y direction is carried out at 115 on the same principle as in Examples 1 and 2, i.e., the concentration in the x, y, and z directions is carried out by flowing the test solution. When the target virus is present in the specimen, it appears as an accumulation of light spots on the microelectrode 115, and the amount of the virus can be measured using the same method as described in the Examples. In Embodiment 1, the electroosmotic flow and dielectrophoresis were performed using different electrodes; however, they may be combined into one electrode. In addition, stirring in the z direction is not performed by the electroosmotic flow but by providing protrusions 125 in the flow path to generate turbulent flow and stirring mechanically or by bringing a vibrator into contact with the detection cell to stir mechanically. Thus, similar effects may be expected (FIG. 11).

Embodiment 2

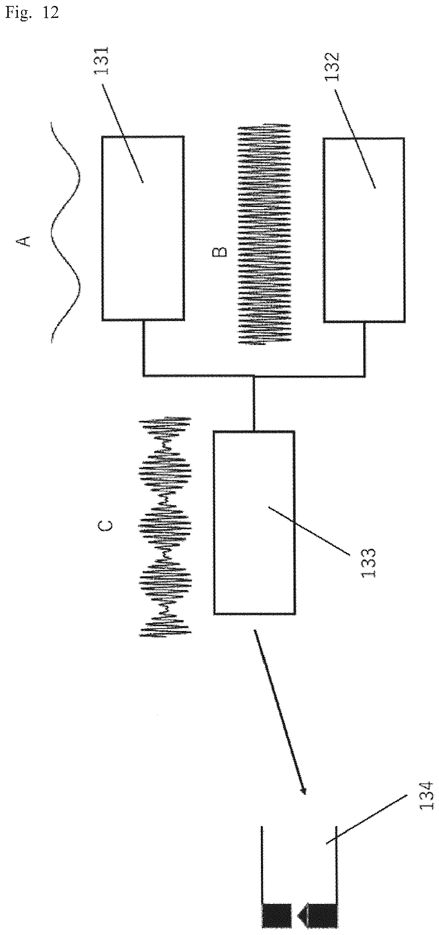

Embodiment 2 will be explained using FIG. 12. In FIG. 12, 131 denotes an AC generator 1, which generates a voltage and frequency (A) suitable for inducing an electroosmotic flow. 132 denotes an AC generator 2, which generates a voltage and frequency (B) suitable for causing dielectrophoresis. 133 denotes a mixer, which combines the alternating current of A and B and generates the alternating current of C. This alternating current of C is applied to the microelectrode 134 provided in the detection cell. Other operations and materials are the same as in Embodiment 1. By adopting this configuration, it becomes possible to apply two types of alternating current to one electrode. By simultaneously performing dielectrophoresis while stirring with the electroosmotic flow, the labeled antibody/NP/RNA complex can be collected on the electrode and observed.

Embodiment 3

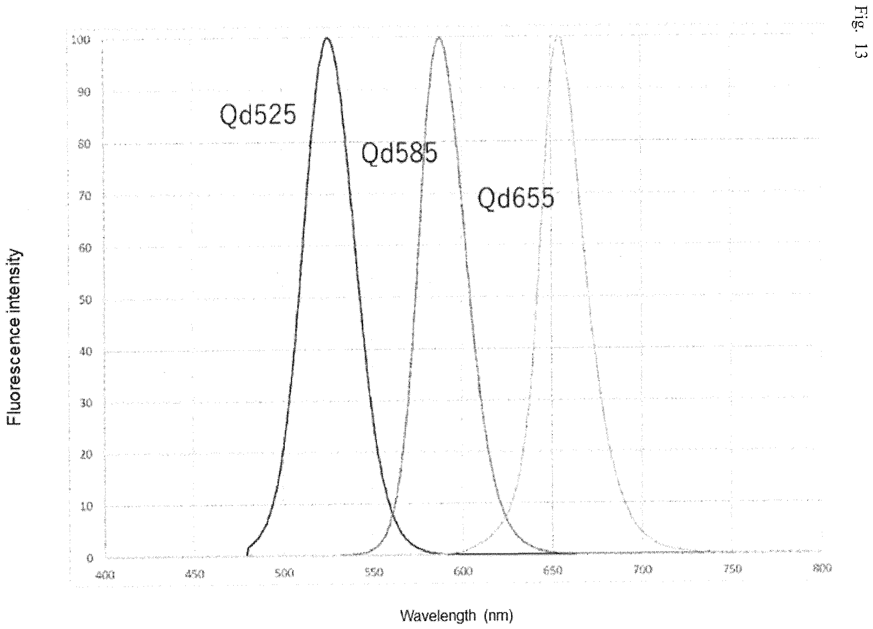

When fluorescence observation is performed, there are no restrictions on the method for labeling an antibody or a fluorescent substance used to label an antibody. However, fluorescence observation is preferably performed using a click reaction, and quantum dots that selectively bind to sugar chains in the Fc region of an antibody are preferably used. A kit with a wide fluorescence wavelength range for quantum dots is available from Thermo Fisher. As an example, FIG. 13 shows fluorescence spectra of three types of quantum dots (Qd525, Qd585, Qd655). As is apparent from FIG. 13, these fluorescence spectra have a narrow half-width of about 30 nm, and, for example, these three types hardly intersect; thus, it is possible to easily obtain signals independently using a color image sensor. Moreover, the excitation wavelength of quantum dots spans the entire wavelength region and is not more than 450 nm. In particular, they emit fluorescence with extremely high efficiency under excitation light of 300 nm or shorter wavelengths. In addition, demand for LEDs that emit so-called deep ultraviolet light around 260 nm for inactivating viruses has increased rapidly recently, and mass production has lowered costs and made them more accessible. By combining these, for example, by labeling the anti-SARS-CoV-2virus NP antibody with Qd525, the anti-influenza A virus NP antibody with Qd585, and the anti-influenza B virus NP antibody with Qd655, it becomes possible to test for three types of infections in one measurement using one specimen.

Reference Signs List

| | |
|---|---|
| 1 | Nucleic acid |
| 2 | Nucleocapsid protein (NP) |
| 3 | Anti-nucleocapsid protein antibody (anti-NP antibody) |
| 4 | Fluorescent substance |
| 5 | Microelectrode |
| 111, 121 | Transparent substrate |
| 112, 122 | Cover glass |
| 113, 123 | Water absorption pad |
| 114, 124 | Composite electrode |
| 115, 125, 134 | Microelectrode for detection |
| 116 | Electrode for electroosmotic flow generation |
| 125 | Protrusions that generate turbulent flow in test solution |
| 131 | Alternating current generator 1 |
| 132 | Alternating current generator 2 |
| 133 | Mixer |

**Claims**

1. A method for detecting a virus in a specimen, comprising:

   a step of bringing a test solution containing the specimen, a surfactant, and a fluorescently labeled anti-nucleocapsid protein antibody into contact with a microelectrode and applying an alternating current voltage to the microelectrode so as to concentrate a fluorescently labeled anti-nucleocapsid protein antibody which is bound to a nucleocapsid protein of the virus in the specimen near the microelectrode; and
   a step of detecting the presence of a virus in the specimen by fluorescence observation,
   wherein the nucleocapsid protein forms a complex with a nucleic acid.

2. The method according to claim 1, wherein the anti-nucleocapsid protein antibody is concentrated near the microelectrode by dielectrophoresis.

3. The method according to claim 1 or 2, wherein an envelope of the virus in the specimen is chemically destroyed by the surfactant.

4. The method according to any one of claims 1 to 3, which further comprises a step of electronically comparing fluorescence image changes in an aggregated state of fluorescence before and after voltage application and converting a difference image into a quantitative value or qualitative judgment result.

5. A virus detection apparatus, comprising:

   a cell having microelectrodes at its bottom;
   a power supply that applies an alternating-current voltage between the microelectrodes;
   an image sensor,
   means for electrically recording images; and
   means for electronically comparing fluorescence image changes in an aggregated state of fluorescence before and after voltage application and converting a difference image into a quantitative value or qualitative judgment result,
   wherein a fluorescently labeled anti-nucleocapsid protein antibody bound to a nucleocapsid protein of a virus in a specimen is concentrated near the microelectrodes so as to carry out fluorescence observation of fluorescence from an anti-nucleocapsid protein antibody.

6. The virus detection apparatus according to claim 5, wherein the fluorescently labeled anti-nucleocapsid protein antibody bound to a nucleocapsid protein of a virus is obtained by chemically destroying an envelope of the virus with a surfactant in a test solution containing a specimen, a surfactant, and a fluorescently labeled anti-nucleocapsid protein antibody.

7. The virus detection apparatus according to claim 5 or 6, wherein a voltage is applied to the test solution such that the fluorescently labeled anti-nucleocapsid protein antibody bound to a nucleocapsid protein of a virus in a specimen is concentrated near the microelectrodes.

8. The virus detection apparatus according to any one of claims 5 to 7, wherein the test solution is stirred by an electroosmotic flow.

9. The virus detection apparatus according to any one of claims 5 to 7, wherein protrusions that generate turbulent flow in a test solution are provided in the cell.

10. The virus detection apparatus according to any one of claims 5 to 9, wherein an alternating current voltage, which can induce an electroosmotic flow and concentrate the fluorescently labeled anti-nucleocapsid protein antibody bound to a nucleocapsid protein of a virus in a specimen, is applied.

11. The virus detection apparatus according to any one of claims 5 to 10, wherein the image sensor is a color sensor with a built-in color filter.

12. The virus detection apparatus according to any one of claims 5 to 11, which is an apparatus for detecting a plurality of viruses simultaneously, wherein a plurality of anti-nucleocapsid protein antibodies fluorescently labeled with different wavelength regions are simultaneously concentrated.

Fig. 1

Equilibrium curve of antigen-antibody reaction

Fig. 2

Fig. 3

Fig. 4

4A

4B

Fig. 5

5A

5B

Fig. 6A

| Time (sec) | Still image | Difference | Quantitative value |
|---|---|---|---|
| 6 | | | 0 |
| 11 | | | 1.171 |
| 16 | | | 1.060 |
| 23 | | | 1.152 |

Fig. 6B

| Time (sec) | Still image | Difference | Quantitative value |
|:---:|:---:|:---:|:---:|
| 39 | | | 0.989 |
| 52 | | | 0.922 |
| 70 | | | 0.939 |
| 85 | | | 0.937 |

Fig. 6C

| Time (sec) | Still image | Difference | Quantitative value |
|:---:|:---:|:---:|:---:|
| 107 | | | 0.948 |
| 133 | | | 0.948 |
| 148 | | | 1.233 |

Fig. 7

Example of digitizing dielectrophoretic images

Fig. 8

Au electrode 20 × Infl.A (add Triton) + Qd585 anti-NP3298 antibody

Fig. 9

Au electrode 20 × Infl.A (add Triton) + Qd585 anti-NP3298 antibody

Fig. 10

Fig. 11

Fig. 12

Fig. 13

## INTERNATIONAL SEARCH REPORT

| International application No. |
| --- |
| **PCT/JP2022/028103** |

### A. CLASSIFICATION OF SUBJECT MATTER

*G01N 33/53*(2006.01)i; *G01N 33/536*(2006.01)i; *G01N 33/569*(2006.01)i; *G01N 21/64*(2006.01)i
FI:   G01N33/569 G; G01N21/64 F; G01N33/536 D; G01N33/53 D

According to International Patent Classification (IPC) or to both national classification and IPC

### B. FIELDS SEARCHED

Minimum documentation searched (classification system followed by classification symbols)

G01N33/53; G01N33/536; G01N33/569; G01N21/64

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X | WO 2021/070884 A1 (NANOTIS CORPORATION) 15 April 2021 (2021-04-15) paragraphs [0016]-[0072], fig. 1-11 | 1-7, 11-12 |
| Y | | 8-10 |
| Y | US 2011/0294225 A1 (WANG, Shau-Chun) 01 December 2011 (2011-12-01) paragraphs [0012]-[0015], [0038]-[0040], fig. 5-7 | 8, 10 |
| Y | US 2018/0149644 A1 (METAL INDUSTRIES RESEARCH AND DEVELOPMENT CENTRE) 31 May 2018 (2018-05-31) paragraph [0028], [0037], fig. 2, 4, 8 | 8, 10 |
| Y | JP 2015-179038 A (TERUMO CORPORATION) 08 October 2015 (2015-10-08) paragraphs [0016], [0017], [0049]-[0053], fig. 4, 5 | 9 |
| Y | JP 2005-151898 A (HITACHI SOFTWARE ENG. COMPANY, LIMITED) 16 June 2005 (2005-06-16) paragraphs [0006]-[0010], [0014]-[0016], fig. 1-5 | 9 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **28 September 2022** | **11 October 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

27

**INTERNATIONAL SEARCH REPORT**

| International application No. |
| --- |
| **PCT/JP2022/028103** |

**C.    DOCUMENTS CONSIDERED TO BE RELEVANT**

| Category* | Citation of document, with indication, where appropriate, of the relevant passages | Relevant to claim No. |
| --- | --- | --- |
| A | JP 2002-174636 A (MATSUSHITA ELECTRIC IND. COMPANY, LIMITED) 21 June 2002 (2002-06-21)<br>        entire text, all drawings | 1-12 |

Form PCT/ISA/210 (second sheet) (January 2015)

**INTERNATIONAL SEARCH REPORT**
**Information on patent family members**

International application No.

**PCT/JP2022/028103**

| Patent document cited in search report | | | Publication date (day/month/year) | Patent family member(s) | | | Publication date (day/month/year) |
|---|---|---|---|---|---|---|---|
| WO | 2021/070884 | A1 | 15 April 2021 | (Family: none) | | | |
| US | 2011/0294225 | A1 | 01 December 2011 | TW | 201144787 | A | |
| US | 2018/0149644 | A1 | 31 May 2018 | TW | 201819904 | A | |
| JP | 2015-179038 | A | 08 October 2015 | (Family: none) | | | |
| JP | 2005-151898 | A | 16 June 2005 | US<br>paragraphs [0007]-[0011],<br>[0024]-[0026], fig. 1-5<br>EP | 2005/0118730<br><br><br>1535666 | A1<br><br><br>A1 | |
| JP | 2002-174636 | A | 21 June 2002 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001165906 A

**Non-patent literature cited in the description**

- **NISHIJIMA.** *SCANS NEWS,* 2005
- **C. K. LEE et al.** *Journal of Clinical Virology,* 2012, vol. 55, 239-243
- **A. SUEKI et al.** *Clinica Chimica Acta,* 2016, vol. 453, 71-74
- *Biochem.J.,* 1983, vol. 211, 281
- **SASAKI.** *Bunseki Kagaku,* 2015, vol. 64 (1), 1